# EUROPÄISCHE PATENTSCHRIFT

(11) **EP 2 018 882 B1**
(45) Veröffentlichungstag und Bekanntmachung des Hinweises auf die Patenterteilung: **31.08.2016**
(21) Anmeldenummer: 08160974.5
(22) Anmeldetag: 23.07.2008
(51) Int. Cl.: A61L 31/02, A61L 31/06, A61L 31/08, A61L 31/10, A61L 31/14

(54) **Endoprothese und Verfahren zur Herstellung derselben**
Endoprosthesis and method for manufacturing the same
Endoprothèse et son procédé de fabrication

(30) Priorität: 24.07.2007 DE 102007034363
(43) Veröffentlichungstag der Anmeldung: 28.01.2009
(73) Patentinhaber: Biotronik VI Patent AG, 6341 Baar (CH)
(72) Erfinder: Klocke, Björn, 8006 Zürich (CH); Adden, Nina, 90427 Nürnberg (DE); Bayer, Ullrich, 76307 Karlsbad (DE)
(74) Vertreter: Galander, Marcus

(56) Entgegenhaltungen:
- EP-A2- 1 752 167
- DE-A1- 10 357 281

## Beschreibung

Die Erfindung betrifft eine Endoprothese oder Implantat, insbesondere eine intraluminale Endoprothese, zum Beispiel einen Stent, mit einem Grundgitter bestehend aus einem im Wesentlichen biodegradierbaren Material und einer auf dem biodegradierbaren Material angeordneten Beschichtung sowie ein Verfahren zur Herstellung einer derartigen Endoprothese.

Stents sind endovaskuläre Prothesen, die zur Behandlung von Stenosen (Gefäßverengungen) eingesetzt werden können. Sie weisen ein rohrförmiges oder hohlzylinderförmiges Grundgitter auf, das an beiden Längsenden offen ist. Das rohrförmige Grundgitter aus dem Grundmaterial einer derartigen Endoprothese wird in das zu behandelnde Gefäß eingesetzt und dient dazu, das Gefäß zu stützen.

Derartige Stents haben sich insbesondere zur Behandlung von Gefäßerkrankungen etabliert. Durch den Einsatz von Stents können verengte Bereiche in den Gefäßen erweitert werden, so dass ein Lumengewinn resultiert. Durch den Einsatz von Stents kann zwar ein für den Therapieerfolg primär notwendiger optimaler Gefäßquerschnitt erreicht werden, allerdings initiiert die dauerhafte Anwesenheit eines derartigen Fremdkörpers eine Kaskade von mikrobiologischen Prozessen, die zu einem allmählichen Zuwachsen des Stents und im schlimmsten Fall zu einem Gefäßverschluss führen können. Ein Ansatzpunkt zur Lösung dieses Problems besteht darin, den Stent aus einem biodegradierbaren Werkstoff zu fertigen.

Unter Biodegradation werden hydrolytische, enzymatische und andere stoffwechselbedingte Abbauprozesse im lebenden Organismus verstanden, die vor allem durch die mit der Endoprothese in Kontakt gelangenden Körperflüssigkeiten verursacht werden und zu einer allmählichen Auflösung zumindest großer Teile der Endoprothese führen. Synonym zu dem Begriff Biodegradation wird häufig der Begriff Biokorrosion verwendet. Der Begriff Bioresorption umfasst die anschließende Resorption der Abbauprodukte durch den lebenden Organismus.

Für das Grundgitter biodegradierbarer Endoprothesen geeignete Werkstoffe (Grundmaterial) können beispielsweise polymerer oder metallischer Natur sein. Das Grundgitter kann auch aus mehreren Werkstoffen bestehen. Gemeinsames Merkmal dieser Werkstoffe ist ihre Biodegradierbarkeit. Beispiele für geeignete polymere Verbindungen sind Polymere aus der Gruppe Cellulose, Kollagen, Albumin, Casein, Polysaccharide (PSAC), Polylactid (PLA), Poly-L-Lactid (PLLA), Polyglykol (PGA), Poly-D,L-Lactid-co-glycolid (PDLLA-PGA), Polyhydroxybuttersäure (PHB), Polyhydroxyvaleriansäure (PHV), Polyalkylcarbonate, Polyorthoester, Polyethylenterephtalat (PET), Polymalonsäure (PML), Polyanhydride, Polyphosphazene, Polyaminosäuren und deren Copolymere sowie Hyaluronsäure. Die Polymere können je nach den gewünschten Eigenschaften in Reinform, in derivatisierter Form, in Form von Blends oder als Copolymere vorliegen. Metallische biodegradierbare Werkstoffe basieren auf Legierungen von Magnesium, Eisen, Zink und/oder Wolfram. Die vorliegende Erfindung bezieht sich vorzugsweise auf Stents oder andere Endoprothesen, deren biodegradierbarer Werkstoff Magnesium oder eine Magnesiumlegierung, besonders bevorzugt die Legierung WE43, und/oder ein biodegradierbares Polymer, besonders bevorzugt PLLA, enthält.

Es sind bereits Stents bekannt, die Beschichtungen mit verschiedenen Funktionen aufweisen. Bei der Realisierung biodegradierbarer Implantate besteht das Problem, die Degradierbarkeit entsprechend der angestrebten Therapie zu steuern. Es konnte bisher noch kein Stent gebaut werden, der im für viele therapeutische Anwendungen wichtigen Zielkorridor von vier Wochen bis sechs Monaten seine Integrität verliert. Hierbei wird unter Integrität, d. h. mechanischer Integrität, die Eigenschaft verstanden, dass der Stent bzw. die Endoprothese gegenüber dem undegradierten Stent kaum mechanische Einbußen besitzt. Dies bedeutet, dass der Stent mechanisch noch so stabil ist, dass der Kollapsdruck lediglich geringfügig, d. h. höchstens auf 80% des Nominalwerts, abgefallen ist. Der Stent kann somit bei vorhandener Integrität seiner Hauptfunktion, dem Aufhalten des Gefäßes, noch nachkommen. Alternativ kann die Integrität dadurch definiert werden, dass der Stent mechanisch so stabil ist, dass er in seinem Belastungszustand in dem Gefäß kaum geometrischen Veränderungen unterworfen ist, beispielsweise nicht nennenswert zusammensackt, d. h. unter Belastung mindestens 80% des Dilatationsdurchmessers, oder kaum angebrochene tragende Streben aufweist.

Für den genannten Zielkorridor der Degradation besonders vielversprechend haben sich degradierbare Magnesium-Stents erwiesen, die allerdings ihre mechanische Integrität bzw. Stützwirkung einerseits noch zu früh verlieren und andererseits in vitro und in vivo einen stark schwankenden Integritätsverlust aufweisen. Dies bedeutet, dass bei Magnesium-Stents der Kollapsdruck über die Zeit zu schnell verringert wird bzw. die Verringerung des Kollapsdrucks eine zu große Variabilität aufweist und damit zu unbestimmbar ist.

Grundsätzlich existieren drei bekannte Ansätze zur Lösung des genannten Problems. Erstens kann ein dickeres, optimiertes Stentdesign gewählt werden. Zweitens kann eine optimierte, langsam degradierende Magnesiumlegierung für den Stent verwendet werden oder drittens können Oberflächenschichten vorgesehen werden, die den Degradationsangriff des Magnesium-Grundgitters verlangsamen bzw. beschleunigen und/oder den Zeitpunkt des Einsetzens der Degradation beeinflussen. Die Möglichkeit, das Degradationsverhalten entsprechend der ersten oder zweiten Lösungsmöglichkeit zu variieren, ist stark eingeschränkt und reicht möglicherweise nicht für eine ökonomisch und klinisch befriedigende Lösung aus. In Bezug auf die erste Lösungsmöglichkeit können nämlich Wandstärken von mehr als 200 µm aufgrund der Gewährleistung einer leichten Einführbarkeit der Stents und der begrenzten Gefäßabmessungen nicht vertreten werden. Für den zweiten Fall ist nur ein sehr beschränktes Spektrum aus biokompatiblen und mäßig schnell degradierbaren Legierungen bekannt. Im Hinblick auf die dritte Lösungsmöglichkeit sind lediglich Passivierungen aus Fluor bekannt.

Die zuvor genannten Passivierungsschichten weisen zwei grundsätzliche Nachteile auf, die sich u. a. dadurch ergeben, dass derartige Stents üblicherweise zwei Zustände annehmen, nämlich einen komprimierten Zustand mit einem kleinen Durchmesser und einen expandierten Zustand mit einem größeren Durchmesser. Im komprimierten Zustand kann der Stent mittels eines Katheters in das zu stützende Gefäß eingeführt und an der zu behandelnden Stelle positioniert werden. Am Ort der Behandlung wird der Stent dann beispielsweise mittels eines Ballonkatheters dilatiert bzw. (bei Verwendung einer Formgedächtnislegierung als Stentmaterial) beispielsweise durch Erwärmung über eine Sprungtemperatur in den expandierten Zustand überführt. Auf Grund dieser Durchmesseränderung ist das Grundgitter des Stents hierbei einer mechanischen Belastung ausgesetzt. Weitere mechanische Belastungen des Stents können während der Herstellung oder bei der Bewegung des Stents im oder mit dem Gefäß, in das der Stent eingesetzt ist, auftreten. Bei den genannten Passivierungen ergibt sich somit der Nachteil, dass während der Verformung des Implantats Mikrorisse entstehen, die zur Unterwanderung des Beschichtungsmaterials führen, so dass die Passivierungswirkung der Beschichtung herabgesetzt wird. Hierdurch wird eine unspezifizierte lokalen Degradation verursacht. Außerdem ist das Einsetzen und die Geschwindigkeit der Degradation von der Größe und der Verteilung der Mikrorisse abhängig, die als Fehlstellen schlecht kontrollierbar sind. Dies führt zu einer starken Streuung in den Degradationszeiten.

In der Druckschrift WO 2005/065576 A1 wird die Degradationssteuerung degradierbarer Implantate durch eine Beschichtung aus einem biodegradierbaren Werkstoff offenbart. Eine ortsabhängige Degradation des Implantats wird dadurch optimiert, dass der Grundkörper eine in vivo ortsabhängige erste Degradationscharakteristik besitzt und eine den Grundkörper vollständig oder ggf. nur bereichsweise bedeckende Beschichtung aus zumindest einem biodegradierbarem Werkstoff aufweist, wobei die Beschichtung in vivo eine zweite Degradationscharakteristik besitzt. Die an einem Ort kumulierte Degradationscharakteristik ergibt sich somit aus der Summe der jeweils an dem genannten Ort bestehenden Degradationscharakteristika von Werkstoff und Beschichtung. Die ortsabhängige kumulierte Degradationscharakteristik wird dabei durch die Variation der zweiten Degradationscharakteristik so vorgegeben, dass die Degradation an dem genannten Ort in einem vorgegebenen Zeitintervall mit einem vorgebbaren Degradationsverlauf stattfindet.

Die in der Druckschrift WO 2005/065576 A1 beschriebene Degradationscharakteristik der biodegradierbaren Beschichtung wird insbesondere durch Variation der morphologischen Struktur der Beschichtung, der stofflichen Modifikation des Werkstoffs und/oder die Anpassung der Schichtdicke der Beschichtung erreicht. Hierbei wird unter morphologischer Struktur die Konformation und Aggregation der die Beschichtung bildenden Verbindungen verstanden.

Die Druckschrift WO 97/11724 A1 beschäftigt sich ebenfalls mit einem biodegradierbaren Implantat und seiner Degradation. Diese Druckschrift offenbart, dass durch Regulierung der makroskopischen Struktur des biodegradierbaren Materials, d. h. zum Beispiel durch unterschiedliche Wanddicken, die Degradation (Disintegration) beeinflusst werden kann. Beispielsweise wird die Wanddicke des Implantats an einem Ende, dem langsamer degradierenden Ende, dicker ausgeführt als an dem anderen, dem schneller degradierenden Ende. Der Druckschrift ist außerdem zu entnehmen, dass die Degradierbarkeit auch durch eine Pre-Hydrolisierung oder eine Änderung der Kristallinität des degradierbaren Materials des Implantats beeinflusst werden kann. Weiterhin wird offenbart, dass mittels einer entsprechenden biodegradierbaren Beschichtung mit einer geringen WasserDurchlässigkeit eine Veränderung des Degradationsverhaltens bewirkt werden kann.

In der US 2006/0224237 A1 wird ebenfalls ein Transplantat oder ein Stent mit einer Schutzschicht beschrieben, das dazu verwendet wird, Oberflächenstrukturen des Stents vor ihrer Zerstörung zu bewahren. Hierbei können die Oberflächenstrukturen aus einem oder mehreren Materialien gebildet werden, die in verschiedenen Umweltbedingungen mindestens teilweise aufgelöst, degradiert oder absorbiert werden.

Die in diesen Druckschriften genannten Möglichkeiten der Beeinflussung der Degradation beinhalten noch keine befriedigenden Lösungen im Hinblick auf Endoprothesen, die in dem genannten Zielkorridor degradieren. In der Druckschrift WO 2005/065576 A1 werden nur sehr allgemeine Prinzipien dargestellt, die keine konkreten Lösungsvorschläge im Hinblick insbesondere auf Magnesium-Stents liefern. Auch die WO 97/11724 A1 bezieht sich vorzugsweise auf Polymerstents. Außerdem ergeben sich durch die Wasserdurchlässigkeit der biodegradierbaren Beschichtung weiterhin Probleme bei der Degradation durch Unterwanderung der Schicht und Gasblasenbildung unter der Deckschicht.

Die Druckschrift US 2007/0050009 A1 beschäftigt sich mit einem Stent, der eine Stützstruktur aus biodegradierbarem Material aufweist. Diese Stützstruktur ist zumindest teilweise mit einer Absorptionsvermeidungsschicht (absorption inhibitor layer) versehen, welche die Rate der Absorption der Stützstruktur verringert. Die Absorptionsvermeidungsschicht selbst wird ebenfalls durch die umgebenden Körperflüssigkeiten absorbiert. Auch mittels dieser bekannten Lösung ist lediglich eine sehr eingeschränkte und für viele Anwendungsfälle ungenügende Steuerung der Degradation des Stents möglich.

EP 1752167 offenbart ein resorbierbares Implantat umfassend einen Grundkörper aus einem vom Körper des Implantatempfängers resorbierbaren Material. Eine titanhaltige Beschichtung bedeckt den Grundkörper teilweise, so dass letzterer beschichtungsfreie Zonen zum Resorptionsangriff des Körpers aufweist.

DE 10357281 offenbart eine insbesondere als Stent ausgeführte degradable Gefäßstütze aus einem Magnesiumwerkstoff. Um einen an den medizinischen Heilungsverlauf angepassten Auflösungsprozess der Gefäßstütze zu ermöglichen, ist die Gefäßstütze zumindest abschnittsweise mit einer die Degradation verzögernden Beschichtung ausgestattet. Beispielsweise können in verschiedenen Bereichen der Beschichtung unterschiedliche Schichtdicken oder Zusammensetzungen erreicht werden, um so über den zeitlichen Verlauf der Degradation eine gezielte Veränderung der Verformungseigenschaften der Gefäßstütze zu erreichen.

Die Aufgabe der vorliegenden Erfindung besteht somit darin, eine Endoprothese anzugeben, deren mechanische Stützwirkung einerseits über einen längeren Zeitraum bestehen bleibt und deren Degradation andererseits zu einem kontrollierbaren Zeitpunkt, insbesondere in dem genannten Zielkorridor stattfindet. Zudem soll die Degradation den geometrischen Gegebenheiten des Stentdesigns und den damit verbundenen klinischen Anforderungen angepasst werden.

Die Aufgabe wird durch eine Endoprothese gelöst, deren Beschichtung inert ausgebildet ist und deren Grundgitter vollständig durch die inerte Beschichtung bis auf mindestens einen Degradationsbereich zur gezielten Steuerung der Degradation in diesem Bereich bedeckt ist, wobei der mindestens eine Degradationsbereich als Aussparung in der inerten Beschichtung ausgebildet ist. Unter Ausparung werden dabei Bereiche in der Beschichtung verstanden, die eine räumliche Ausdehnung von rund 300 nm bis maximal fast zur Ausdehnung der Endoprothese (z.B. 10mm) aufweisen und die das Grundgitter freilegen und es somit es den bei der Degradation beteiligten Molekülen, zumindest Wasser, erlauben, das Grundgitter zu erreichen. Dabei ist eine Vielzahl von Degradationsbereichen lediglich im Bereich der Verbindungsstege angeordnet.

Unter einer inerten Beschichtung wird eine Schicht verstanden, die mit der jeweiligen Umgebung des behandelten Körpers (physiologische Umgebung mit einem physiologischen pH-Wert) im Wesentlichen weder chemisch noch biologisch wechselwirkt, d.h. von dieser Umgebung im Wesentlichen nicht absorbiert wird, und eine Diffusion von Wasser oder anderen Molekülen nahezu vollständig unterbindet und somit im Wesentlichen dicht gegenüber diesen Molekülen ist. Eine weitere Eigenschaft des Beschichtungsmaterials besteht darin, dass es nicht nennenswert quillt. Eine Degradation des darunter liegenden biodegradierbaren Materials wird durch die inerte Beschichtung folglich ebenfalls verhindert. Zudem ist inertes Material nicht thrombogen und weist keinen negativen bzw. pathologischen Einfluss auf das umliegende Gewebe bzw. die umgebende Körperflüssigkeit auf.

Die erfindungsgemäße Endoprothese weist demnach Degradationsbereiche in der Form von geometrisch kontrollierten Aussparungen auf, die ganz gezielt lediglich einen Teil der Oberfläche des Endoprothesen-Grundgitters freigeben bzw. leichter angreifbar machen. Dort degradiert das degradierbare Material direkt im Kontakt zum Körpergewebe und Körperflüssigkeit, wie z.B. Blut, schneller. Idealerweise ist die Geometrie der Aussparungen, d.h. deren geometrische Form so gewählt, dass die lokale Degradation geordnet und vorhersehbar abläuft. Somit kann der gewünschte Integritätsverlust auf die gewünschte Zeit, insbesondere vier Wochen bis sechs Monate, eingestellt werden.

Die Aussparungen lassen sich bei der Herstellung der Endoprothese vorzugsweise durch Verwendung von Schablonen oder mechanischen Kontakten bei der Beschichtung erzeugen. Um zum Beispiel einen Stent komplett mit Ausnahme der luminalen Seite zu beschichten, kann der Stent unter leichter Eigenspannung auf einen zylindrischen Körper (Innendorn) geschoben werden, so dass die Stent-Innenseite während des Beschichtungsvorgangs nicht in Kontakt mit dem Beschichtungsmittel kommt. Durch eine geeignete Oberflächenstrukturierung des Innendorns lässt sich dieser am Ende des Beschichtungsvorgangs wieder aus dem Stent ziehen.

Ausgehend von dem Degradationsbereich wird bei der Degradation einer erfindungsgemäßen Endoprothese das degradierende Endoprothesenmaterial ausgeschwemmt und es bleibt ein dünner Schlauch aus der inerten Beschichtung ggf. Abbauprodukte des degradierten Endoprothesenmaterials enthaltend bestehen. Bei einer Endoprothese aus einer Magnesium-Legierung entstehen beispielsweise weiche Magnesiumabbauprodukte bzw. - umbauprodukte wie Calciumphosphat (aus dem körpereigenen Puffersystem), sowie ggf. Magnesiumhydroxid oder auch Magnesiumphosphat. Das Auftreten von solchen Produkten ist bei der Auswahl geeigneter biokompatibler Legierungen klinisch akzeptabel.

Die inerte Beschichtungen weist beispielsweise ein oder mehrere Polymere der Gruppe bestehend aus Polyphosphazene, Silicone, Polyolefine, Polyisobutylen, Vinylhalogenid Polymere und Copolymere, wie Polyvinylchlorid, Polyvinylether, wie Polyvinylmethylether, Polyvinylketone, Polyvinylaromaten, wie Polystyrol oderPoly(styrol-isoprene-styrol), Polyvinylester, wie Polyvinylacetat, Copolymere von Vinyl Monomeren miteinander und Olefinen, wie Ethylen, Methylmethacrylat Copolymere, Acrylnitril-Styrol Copolymere, Polysulfon, Poly(n-butylmethacrylat), Poly(sec-butylmethacrylat), Poly(isobutylmethacrylat), Poly(tert-butylmethacrylat), Poly(n-propylmethacrylat), Poly(isopropylmethacrylat), Poly(ethylmethacrylat), Poly(methylmethacrylat), Polyurethane, Polyisobutylenpoly(methylmethacrylat) und Polydimethylsiloxan auf.

Die inerte Beschichtung kann auch derart gestaltet sein, dass sie nach der Degradation des Grundgitters in der basischen Umgebung, d.h. einer Umgebung, die einen höheren pH-Wert aufweist als die physiologische Umgebung und die durch die Abbauprodukte des Endoprothesenmaterials entsteht, degradiert. Hierbei müssen die Abbauprodukte der pHsensitiven Beschichtung biokompatibel sein und lediglich in einer geringen Menge auftreten. Beispielsweise degradieren alle Mg-Legierungen unter Bildung von Mg-Ionen basisch. Beim Abbau von Magnesiumstents in den Oberflächenschichten werden z.B. pH-Werte von 10 bis 11 erreicht. Für eine derartige Beschichtung geeignete Polymere sind spezifische Polyester (zum Beispiel ausgewählte Polylactide, beispielsweise Poly-L-Lactid (PLLA)) und Polypeptide, welche bei den genannten pH-Werten alkalisch aufgebrochen werden.

In einem bevorzugten Ausführungsbeispiel ist die Beschichtung zusätzlich flexibel ausgebildet. Hierbei bedeutet das Merkmal, dass die Beschichtung flexibel ausgebildet ist, dass die Beschichtung die Bewegung des Grundgitters nachvollzieht, so dass sich im Wesentlichen keine größeren Risse oder dergleichen in dem Beschichtungsmaterial ausbilden. Das bedeutet, dass das Material der Beschichtung selbst keine Stützfunktion aufweist, d.h. dass die Beschichtung elastisch und somit biegbar ausgebildet ist. Dies tritt umso mehr zutage, je mehr das Grundgitter, das unter der Beschichtung angeordnet ist, aufgrund der Degradation in den Degradationsbereichen degradiert, wobei die Degradation sich auch - von einem seitlich angeordneten Degradationsbereich kommend - unterhalb der inerten Beschichtung fortsetzt. Nach vollständiger Degradation bewegt sich die flexible Beschichtung, die selbst keine Stützfunktion hat, flexibel mit dem behandelnden Gefäß, in dessen Wand sie typischerweise durch Endothelialisierung und teilweise auch Neointimaproliferation eingebettet ist, und der darin strömenden Körperflüssigkeit.

In einem weiteren bevorzugten Ausführungsbeispiel weist die inerte Beschichtung eine oder mehrere Verbindungen der Gruppe bestehend aus Polysulfon, Silikonkautschuk, Polyurethan, Diamond Like Carbon (DLC) und synthetisches Glycocalix auf. Diese Materialien eignen sich besonders gut als flexible inerte Beschichtung und sind kostengünstig aufbringbar. Neben der genannten Gruppe von speziellen organischen Stoffen eignen sich auch amorphes Siliziumcarbid, Magnesiumphosphat sowie Magnesiumoxid und deren Mischverbindungen als besonders bevorzugte inerte, aber nur wenig flexible Beschichtungsmaterialien.

Die obige Aufgabe wird ferner durch eine Endoprothese gelöst, wobei die auf der Oberfläche des Grundgitters angeordnete Beschichtung Parylene, vorzugsweise mindestens überwiegend Parylene, besonders bevorzugt Parylene C oder Parylene N, enthält, wobei das Grundgitter vollständig durch die Beschichtung bis auf mindestens einen Degradationsbereich zur gezielten Steuerung der Degradation in diesem Bereich bedeckt ist, wobei der mindestens eine Degradationsbereich als Aussparung in der Beschichtung ausgebildet ist. Insbesondere bei einer Beschichtung mit Parylene, höchstvorzugsweise einer Beschichtung, welche zu mindestens 30 Gew.% aus Parylene, bevorzugt aus Parylene C und/oder Parylene N, besteht wird das Degradationsverhalten besonders positiv beeinflusst. Parylene weist insbesondere die oben beschriebenen Eigenschaften Flexibilität und geringes Quellvolumen auf. Dabei ist eine Vielzahl von Degradationsbereichen lediglich im Bereich der Verbindungsstege angeordnet.

Als Parylene werden vollständig lineare, teilkristalline und unvernetzte aromatische Polymere bezeichnet. Diese Polymere lassen sich, je nach ihrem Aufbau, in vier verschiedene Grundtypen einteilen, nämlich Parylene C, Parylene D, Parylene N und Parylene F.

Parylene ist bevorzugt mittels eines Plasmabeschichtungsverfahrens aufbringbar. In einem bevorzugten Ausführungsbeispiel beträgt die Dicke der Beschichtung mit Parylene, vorzugsweise der Beschichtung von Parylene C oder Parylene N, in den von den Degradationsbereichen verschiedenen Bereichen zwischen etwa 0,1 µm und etwa 10 µm, bevorzugt zwischen etwa 0,4 µm und etwa 7 µm, besonders bevorzugt zwischen etwa 1 µm und etwa 5 µm. Bei einer Schichtdicke oberhalb von 10 µm wird die Beschichtungszeit zu lang, so dass das Beschichtungsverfahren zu kostenaufwendig wird. Außerdem führt eine Beschichtung mit einer großen Dicke zu einer nennenswerten Verringerung des blutdurchflossenen Lumens im Gefäß des Behandelten (u.a. auch durch die induzierte Neointimabildung). Bei einer Schichtdicke von weniger als 0,1 µm bilden sich in der Beschichtung Inhomogenitäten bezogen auf die Dicke der Schicht sowie Fehlstellen. Hierdurch kann die Degradation des darunter liegenden Endoprothesen-Grundgitters nicht mehr zuverlässig verhindert werden bzw. die Degradation verläuft mit einer zu großen und unerwünschten Variabilität.

Während vollständig geschlossene Parylene-Schichten erfindungsgemäß nachträglich mit Aussparungen versehen werden müssen, kann Parylene auch in so dünnen Schichten (je nach Material und Beschaffenheit der Grundgitteroberfläche mit einer Dicke von etwa 0.1 µm bis 1 µm) aufgebracht werden, dass die Schicht nicht geschlossen ist, sondern sich noch in einer Phase des Inselwachstums befindet, wobei die Aussparungen aus den nicht bzw. kaum beschichteten Oberflächenanteilen gebildet werden.

In den mit der inerten und vorzugsweise flexiblen Beschichtung oder der Parylene enthaltenden Beschichtung versehenen Bereichen wird die Oberfläche der Endoprothese von der inerten und biokompatiblen Deckschicht derart geschützt, dass sie im Gegensatz zu bekannten Passivierungen die mechanischen Beanspruchungen wie Crimpen, Dilatieren oder Crossing der Lesion übersteht, ohne dass sich Risse oder andere Fehlstellen ausbilden. Hierdurch wird eine unkontrollierte Degradation der Endoprothese an Stellen, an denen diese nicht gewünscht ist, verhindert. Durch die Vermeidung der Riss- oder Fehlstellenbildung bei einer flexiblen Beschichtung kann eine starke Streuung der Degradationszeiten verhindert werden. In einem bevorzugten Ausführungsbeispiel sind die Degradationsbereiche in Form von Aussparungen so angeordnet, dass die Rissausbreitung wesentlich behindert wird.

Die Degradationsbereiche können auf verschiedene Weise hergestellt werden.

Beispielsweise kann Laserstrahlung zur lokalen Ablation der Beschichtung durch thermische Strahlung im infraroten Wellenbereich verwendet werden. Hierbei eignen sich vor allem Yb:YAG-, Nd:YAG- oder CO₂-Laser sowie Femtosekunden-Laser oder Faserlaser zur Bearbeitung.

Ferner kann Elektronenstrahlung eingesetzt werden, um lokal thermisch die chemischen oder physikalischen Eigenschaften der Beschichtung zu ändern. Die Elektronenstrahlquelle wird bevorzugt in einer Vakuumkammer und besonders bevorzugt mit einer Scaneinheit mit Multispoteinrichtung verwendet. Die Scaneinheit mit Multispoteinrichtung ermöglicht eine quasisimultane Erwärmung der Endoprothese an mehreren Stellen. Zur thermischen Modifikation der Beschichtung können ebenso elektromagnetische Wechselfelder eingesetzt werden.

Wie bereits oben angegeben wurde, ist insbesondere eine Parylene enthaltende Beschichtung vorzugsweise mittels eines Plasmabeschichtungsverfahrens aufbringbar. Es können sowohl Gas- als auch elektrolytische Plasmaprozesse verwendet werden. Hierbei muss das Plasma mit geeigneten technischen Einrichtungen (Abschirmung, Gasstrom, Gegenelektrodenform etc.) gezielt zur Bearbeitung einzelner Geometriesegmente der Endoprothese eingestellt werden. Ggf. kann die luminale Seite bei der Durchführung des Plasmabeschichtungsverfahrens z.B. durch Aufziehen auf einen Silikonschlauch abgeschattet werden. Bei diesem Verfahren wird während der Beschichtung der Silikonschlauch teilweise unterwandert.

Ionenstrahlung kann ebenfalls zur Modifikation der Beschichtung eingesetzt werden. Lokal können die Degradationsbereiche auch durch eine Bestrahlung mit flüchtigen Festkörpern (z. B. Trockeneis) behandelt werden, so dass die Beschichtung an den behandelten Stellen lokal versprödet. Anschließend können die Schichtbereiche mittels anderer Prozesse, beispielsweise mittels Laser-, Elektronen- oder Ionenbestrahlung entfernt werden. Die Degradationsbereiche können auch durch eine Bestrahlung mit Festkörpern (Sand, Keramik, Magnesium, Salze usw.) oder Flüssigkeiten (Wasserstrahl, Öle, Säure, Fette) oder Festkörper-/Flüssigkeitsgemischen erzeugt werden. Ebenso können Degradationsbereiche durch mechanische Bearbeitung der Schicht (beispielsweise mittels Nadeln, Bürstensystemen) in Trommeln beziehungsweise durch Gleitschleifverfahren (Trowalisieren) hergestellt werden.

Bei den vorgenannten Herstellungsverfahren können entsprechende Optiken, welche an die entsprechende Endoprothesengeometrie angepasst sind, verwendet werden. Im Fall der Laserbestrahlung können Faseroptiken verwendet werden. Weiter können hochdynamische Handhabungstechniken eingesetzt werden und mittels Masken Bereiche der beschichteten Endoprothesenoberfläche, welche nicht bearbeitet werden soll, abgeschirmt werden.

Bei einem besonders bevorzugten Herstellungsverfahren einer Parylene enthaltenden Beschichtung mit Degradationsbereichen werden diese durch ein sich an das Aufbringen der Beschichtung anschließende Ätzen in einem Sauerstoffplasma erzeugt. Beschichtungen mit Parylene des Typs C und N führen in der Regel zu einer makroskopisch gleichmäßigen Bedeckung der Oberfläche der Endoprothese. Mikroskopisch existieren bei beiden Schichtvarianten jedoch verteilt über die Oberfläche der Endoprothese Schichtdickenunterschiede im Bereich einiger 0,1 µm.

Wird eine mit einer Parylene enthaltenden Beschichtung von bevorzugt 1 bis 5 µm Dicke bedeckte Oberfläche einer Endoprothese einer Sauerstoffplasmabehandlung unterzogen, so wird die Beschichtung durch die Sauerstoff-Ionen attackiert. Dies hat eine örtlich selektive Verringerung der Parylene enthaltenden Beschichtung zur Folge. Dieser Verlust an schützender Wirkung der Beschichtung ist umgekehrt proportional zur Schichtdicke. Werden nun die Verfahrensparameter (z.B. Sauerstoffpartialdruck, Einwirkzeit, Kammertemperatur) des Sauerstoffplasmas so gesteuert, dass an ausgewählten Schwachstellen der Beschichtung diese bis zum Grundwerkstoff oxidiert wird, so entstehen örtlich begrenzte Pinholes (Aussparungen), die frei von der Beschichtung sind und eine Ausdehnung von nur wenigen µm² Flächeninhalt aufweisen. Die Oberfläche dieser Pinholes besteht fast ausschließlich aus dem Oxid des Endoprothesen-Grundwerkstoffs, vorzugsweise Magnesiumoxid. Die Oberfläche der Pinholes zeichnet sich im Vergleich zu den unter atmosphärischen Bedingungen entstandenen Oberfläche des Endoprothesen-Grundmaterials durch weitgehendes Fehlen von Hydroxid aus. Dadurch weisen die Pinholes eine Korrosionsbeständigkeit auf, die geringer als die der Beschichtung, aber größer als eine unbehandelte Endoprothesen-Oberfläche ist. Dadurch findet der beim späteren Einsatz der Endoprothese unter Körpermilieubedingungen an diesen Pinholes einsetzende Degradationsangriff zunächst verzögert statt und führt nach einer teilweisen Umwandlung des Oxides zu Hydroxid zur Korrosion des Grundmaterials. Da nahezu alle Pinholes einer solchen Endoprothese eine identische Oberflächenzusammensetzung aufweisen, werden diese Stellen gleichmäßig korrosiv beansprucht. Eine solche gleichmäßige und verzögerte lokale Degradation stellt die Grundlage einer kalkulierbaren integralen Endoprothesendegradation dar. Zum Ätzen der Beschichtung können analog die Verfahren Plasmaätzen, Reactivelon-Etching und Deep-Reactive-Ion-Etching angewendet werden.

Als weitere Möglichkeit kann auf die Beschichtung auch ein Resist aufgebracht werden. Dieser Resist wird so strukturiert, dass gezielt nur die Beschichtung an bestimmten Stellen (Sollbruchstellen) abgetragen wird, und wird nachträglich nasschemisch wieder abgelöst.

Als weitere Herstellungs-Variante ist eine spezielle Formgebung/Bearbeitung von degradierberen Endoprothesen möglich, die Schwachstellen in der später aufgebrachten Parylene-Schicht erzeugt. In dieser Variante entsteht die erfindungsgemäßen Aussparungen noch nicht bei der eigentlichen Produktion der Endoprothese sondern erst im Laufe der Implantation dieser. Diese Schwachstellen (Sollbruchstellen) werden z.B. durch Löcher bzw. Makroporen in den Stegen eines Stents realisiert, die mittels Laserschneiden eingebracht werden. Dieser Verfahrensschritt wird im Verlauf des üblichen Laserschneidprozesses zur Anwendung gebracht. Die nachfolgende Parylene-Beschichtung führt zwar zunächst zu einem auch diese Sollbruchstellen versiegelnden Effekt. Allerdings kommt es bereits beim Dilatieren des Stents zur Mikrorissbildung bevorzugt in der Nähe der Zonen mit den Sollbruchstellen. Diese Mikrorisse treten vor allem in den sich durch höchste Spannungskonzentrationen auszeichnenden Gebieten um die Sollbruchstellen auf. Dort findet dann der bevorzugte Korrosionsangriff statt. Dieser zeichnet sich dadurch aus, dass er zeitlich gleichmäßig und gleich stark in den Zonen um die Sollbruchstelle stattfindet. Das Korrosionsmedium dringt in die mikrorissbehaftete Parylene-Schicht ein, korrodiert das darunter angeordnete degradierbare Material des Grundgitters und führt letztendlich zu einer wochengenau kalkulierbaren Querschnittsschwächung der Stentstege durch Korrosionsangriff. Alternativ entstehen in der Parylene-Schicht Sollbruchstellen an den Teilen des Stents, die sich schon konstruktiv bedingt an der Oberfläche durch Crimpen und Aufdilatieren stark verformen.

In einem weiteren Ausführungsbeispiel der Erfindung ist eine Haftvermittlerschicht zwischen der inerten Beschichtung und dem Material des Grundgitters vorgesehen, die nicht im Bereich der Aussparungen des Degradationsbereichs angeordnet ist. Eine derartige Haftvermittlerschicht verbessert die Haftung zwischen der inerten Beschichtung und dem Material des Grundgitters. Eine derartige Haftvermittlerschicht kann beispielsweise eine oder mehrere der Verbindungen aus der Gruppe Magnesiumoxid, Magnesiumphosphat, anorganische Magnesium-Verbindungen aufweisen.

Bei einem Aufbau der Endoprothese aus Stützelementen, die vorzugsweise zick-zack-, mäander- oder spiralförmig ausgebildet sind und die Abstützung des Gefäßes oder anderer Hohlorgane übernehmen, und aus diese Stützelemente verbindenden Verbindungsstegen, die selbst keine stützende Funktion übernehmen, ist eine Vielzahl von Degradationsbereichen lediglich im Bereich der Verbindungsstege angeordnet. Beispielsweise wird je eine Aussparung lediglich in der Mitte eines Verbindungsstegs angeordnet. Eine derartige Ausführungsform ist besonders einfach aufgebaut und kann außerdem mit geringen Produktionskosten realisiert werden. Eine derartige Endoprothese hat den Vorteil, dass ihr Kollapsdruck nach einem gewünschten Zeitraum wie 4 Wochen bis 6 Monate sehr schnell abfällt. Eine solche Endoprothese ist für den klinischen Einsatz besonders gewünscht.

Vorzugsweise ist an dem Grundgitter mindestens ein Degradationselement vorgesehen, das von dem Grundgitter im Wesentlichen fortsatzartig wegragt und mindestens einen Degradationsbereich aufweist. Besonders einfach herstellbar sind derartige Degradationselemente, wenn sie im Wesentlichen fingerförmig ausgebildet sind. Ein Degradationselement kann jedoch auch eine andere Form aufweisen. Ein derartiges Degradationselement weist vorzugsweise außer der Aufgabe, die Degradation zu steuern, keine weitere Funktion auf, insbesondere hat das Degradationselement keine mechanische Funktion im Hinblick auf die Endoprothese. Das Degradationselement ist erfindungsgemäß vorzugsweise aus dem gleichen Material wie das Grundgitter ausgebildet und weist ebenfalls die vollständige Beschichtung aus dem flexiblen inerten Material mit der gleichen Schichtdicke wie an dem Grundgitter auf außer in dem oder den Degradationsbereich(en). Hierdurch kann das Degradationselement als Teil des Stents einfach zusammen mit dem Grundgitter, beispielsweise durch Schneiden mittels Laser hergestellt werden. Das Degradationselement bildet somit gewissermaßen eine (vorzugsweise nicht mechanisch tragende) Zündschnur, bei der die Degradation beginnt, so dass die Degradation für eine je nach Ausbildung des Degradationselements steuerbare Zeit von den funktionellen Elementen der Endoprothese ferngehalten wird.

Weiter bevorzugt erstreckt sich das Degradationselement im Wesentlichen in dem Bereich des durch das Grundgitter ausgebildeten Mantelvolumens. Dieses Mantelvolumen ist der äußere Mantelbereich des von der Endoprothese eingenommenen Zylinders. Wenn das Degradationselement nicht nach innen radial aus diesem Mantelbereich herausragt, werden zusätzliche, unerwünschte Turbulenzen in der Körperflüssigkeit, die in dem mit der Endoprothese versehenen Gefäß fließt, vermieden. Auch ein Wegragen des Degradationselements nach außen ist nicht erwünscht, da sonst das Degradationselement das Gefäß oder Hohlorgan, in dem die Endoprothese angeordnet ist, durchdringen würde.

In einem besonders bevorzugten Ausführungsbeispiel weist jedes Degradationselement genau einen Degradationsbereich auf, der an dem Ende des Degradationselements angeordnet ist, das von dem Grundgitter wegragt. Dies bedeutet, dass beispielsweise das von dem Grundgitter wegragende Ende keine inerte Beschichtung aufweist und somit freiliegt. Hierdurch beginnt die Degradation an diesem Ende des Degradationselements. Durch die Dicke und Länge sowie die Anordnung des Degradationselements am Grundgitter kann gesteuert werden, wann und an welcher Stelle anschließend das Grundgitter degradiert.

Um die gewünschten Degradationszeiten im Zielkorridor von vier Wochen bis sechs Monaten zu erreichen, weisen die Degradationselemente in einem bevorzugten Ausführungsbeispiel einen Durchmesser von etwa 50 bis etwa 200 µm auf. Im Falle eines Stents ist es fertigungstechnisch bevorzugt, wenn die Degradationselemente die gleiche Geometrie bzw. Dicke wie die Stentstreben aufweist. Bevorzugt ist ebenfalls, wenn die Degradationselemente eine Länge von bis zu etwa 0,3 mm, vorzugsweise von etwa bis zu 0,1 mm haben. Dabei wird die Länge der Degradationselemente und ihre Dicke ohne Berücksichtigung der inerten Beschichtung gemessen. Bei der Wahl der Abmessungen der Degradationselemente ist wichtig, dass sich die Degradationselemente auch beim gecrimpten Stent auf dem Katheter die Stentstreben nicht berühren. Hierdurch wird vermieden, dass die Beschichtung beim Crimpen beschädigt (abgekratzt) wird.

In einem weiteren bevorzugten Ausführungsbeispiel ist der Degradationsbereich als ringförmige Aussparung ausgebildet, die sich um ein Stützelement, einen Verbindungssteg oder ein Degradationselement herum erstreckt. In weiteren Ausführungsbeispielen kann die Aussparung auch andere Formen aufweisen, die sich vollständig oder teilweise um die genannten Elemente herum erstrecken. In einem weiteren bevorzugten Ausführungsbeispiel ist der Degradationsbereich als kreisrunde, mehreckige oder als mit jeder denkbaren anderen Form versehene Aussparung ausgebildet. Besonders bevorzugt weisen derartige Aussparungen einen Durchmesser von etwa 1 bis 10 µm auf. Derartige Ausgestaltungen von Degradationsbereichen lassen sich besonders einfach und kostengünstig herstellen. Bevorzugt sind weiterhin Aussparungen mit geraden Kanten.

Besonders bevorzugt ist mindestens ein Degradationsbereich an einer Vielzahl in einem vorgegebenen Bereich der Endoprothese angeordneten Verbindungssteg und/oder an den an einem dieser Verbindungsstege angeordneten Degradationselementen vorgesehen. Hierbei bilden die Verbindungsstege zusammen mit den Stützelementen das Grundgitter. Das Grundgitter besteht aus in axialer Richtung hintereinander angeordneten, im Wesentlichen in Umfangsrichtung verlaufenden Stützelementen und die einzelnen Stützelemente verbindenden Verbindungsstegen. Hierdurch werden die Verbindungsstege zuerst degradiert, so dass zeitnah nach dem Einsetzen der Endoprothese die Flexibilität im Hinblick auf das Gefäß optimiert wird. Die Integrität der stützenden ringförmigen Stützelemente wird länger, d. h. so lange aufrecht erhalten, wie dies klinisch notwendig ist.

Die Erfindung wird nachfolgend anhand von mittels Figuren dargestellten Ausführungsbeispielen näher erläutert. Dabei bilden alle beschriebenen und/oder bildlich dargestellten Merkmale den Gegenstand der Erfindung, unabhängig von ihrer Zusammenfassung in den Ansprüchen oder deren Rückbeziehung.

Es zeigen schematisch:
- Figur 1: einen Abschnitt eines ersten Ausführungsbeispiels einer erfindungsgemäßen Endoprothese in einem Querschnitt,
- Figur 2: einen Abschnitt eines weiteren Ausführungsbeispiels einer erfindungsgemäßen Endoprothese in einem Querschnitt,
- Figur 3: einen Abschnitt eines dritten Ausführungsbeispiels einer erfindungsgemäßen Endoprothese in einer Ansicht von der Seite,
- Figur 4: die Strukturformeln von Parylene C (Figur 4a) und Parylene N (Figur 4b) sowie
- Figur 5: einen Abschnitt eines vierten Ausführungsbeispiels einer erfindungsgemäßen Endoprothese in einer Ansicht von der Seite.

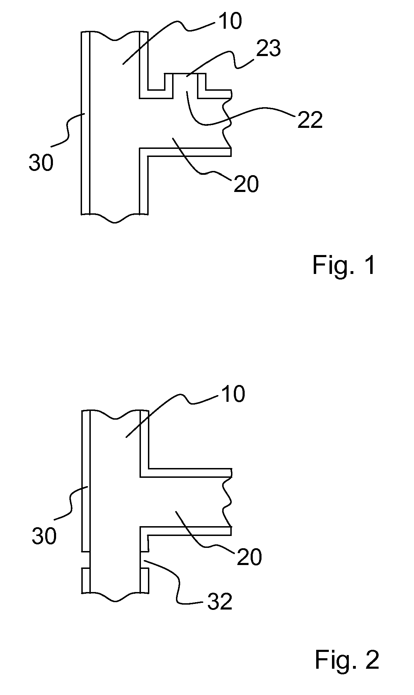

Figur 1 zeigt einen Abschnitt eines Grundgitters einer erfindungsgemäßen Endoprothese, die als Stent ausgebildet ist. Das Grundgitter weist zick-zack- oder mäanderförmig gefaltete, im Wesentlichen in Umfangsrichtung verlaufende oder helixförmige Stege als Stützelemente 10 sowie im Wesentlichen in Längsrichtung des Stents verlaufende Stege als Verbindungsstege 20 auf. Der Stent ist insgesamt als eine in Richtung der Verbindungsstege 20 verlaufende rohrförmige oder hohlzylinderförmige Endoprothese, die an ihren Enden offen ist, ausgebildet. In Figur 1 ist lediglich ein Abschnitt des Grundgitters dargestellt, in dem das Ende eines Verbindungsstegs 20 an ein Stützelement 10 stößt.

Das Grundgitter des Stents besteht zumindest überwiegend aus einem oder mehreren weitestgehend biodegradierbaren Materialien und weist auf seiner gesamten Oberfläche außer in den unten im Einzelnen beschriebenen Degradationsbereichen eine das Grundgitter vollständig bedeckende inerte und flexible Beschichtung oder eine Parylene enthaltende Beschichtung 30 mit einer im Wesentlichen konstanten Schichtdicke auf. Besonders gebräuchliche biodegradierbare Materialien sind oben angegeben. Als Materialien für die Beschichtung 30 kommen beispielsweise Parylene C oder Parylene N oder Polysulphon in Frage.

Das in Figur 1 dargestellte Ausführungsbeispiel weist an einer Vielzahl von Verbindungsstegen 20 ein Degradationselement 22 in der Form eines fingerförmigen Fortsatzes auf. Der fingerförmige Fortsatz 22 hat eine im Wesentlichen zylindrische Form, die sich in einem weiteren, nicht dargestellten Ausführungsbeispiel in die von dem Grundgitter weg weisende Richtung verjüngen, d. h. ihren Durchmesser verringern, kann.

An seinem von dem Verbindungssteg 20 wegragenden Ende 23 weist das Degradationselement 22 keine Beschichtung 30 auf. Hier liegt das Material des Stents frei, so dass die Körperflüssigkeit direkt an dem freiliegenden degradierbaren Material des fingerförmigen Fortsatzes angreifen kann und eine Degradation dieses Materials bewirkt. Das Ende 23 des fingerförmigen Fortsatzes 22 ist somit ein Degradationsbereich, an dem die Degradation des Stents beginnt. In den übrigen Bereichen verhindert die Beschichtung 30 die Degradation.

Das in Figur 2 dargestellte Ausführungsbeispiel eines erfindungsgemäßen Stents weist an dem Teil des dargestellten Verbindungsstegs 20 keinen fingerförmigen Fortsatz 22 auf. Stattdessen ist im Bereich des Stützelements 10 eine ringförmige, um das Stützelement 10 herum laufende Aussparung 32 vorgesehen, so dass ebenfalls das degradierbare Material des Stützelements 10 für einen Degradationsangriff frei liegt. Auch durch eine derartige Aussparung kann genau gesteuert werden, wo die Degradation des erfindungsgemäßen Stents beginnt.

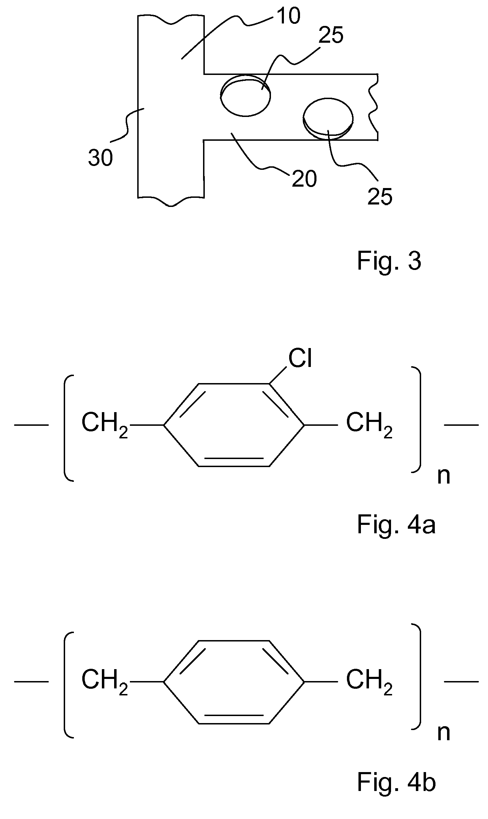

In dem anhand von Figur 3 dargestellten dritten Ausführungsbeispiel sind die Degradationsbereiche durch vorzugsweise an den Verbindungsstegen 20 angeordnete kreisrunde Bereiche 25 realisiert, in denen die Beschichtung 30 gänzlich ausgespart ist und das degradierbare Material freiliegt. Auch an diesen Stellen wird die Degradation des Stents nach dem Einsetzen in den Körper beginnen.

Die gezeigten Ausführungsbeispiele für die Anordnung der Degradationsbereiche können beliebig entsprechend des gewünschten Degradationsverhaltens variiert werden. Demzufolge können die fingerförmigen Fortsätze 22 auch an den Stützelementen 10 oder an anderen Stellen der Verbindungsstege 20 angeordnet sein. Zudem können die fingerförmigen Fortsätze 22 auch mehrfach an den Stützelementen oder lediglich an bestimmten Stützelementen 10 bzw. Verbindungsstegen 20 angeordnet sein. Gleiches gilt auch für die ringförmige Ausnehmung 32 oder den kreisrunden Bereich 25. Die verschiedenen Typen der Degradationsbereiche können zudem beliebig (auch mit den Degradationselementen) an einer Endoprothese kombiniert werden.

Die Endoprothesen können dadurch hergestellt werden, dass zunächst die Endoprothese mit den bekannten Herstellungsverfahren aus dem biodegradierbaren Material hergestellt wird. Ggf. sind hierbei an dem Grundgitter an den gewünschten Stellen die fingerförmigen Fortsätze 22 oder andere Degradationselemente vorzusehen. Anschließend wird die Beschichtung 30 mittels bekannter Beschichtungsverfahren (zum Beispiel für Parylene mittels eines Plasmabeschichtungsverfahrens) aufgebracht, wobei an den Stellen, an denen ein Degradationsbereich vorgesehen werden soll, vor der Beschichtung eine Abdeckung angeordnet wird, so dass die Beschichtung während des Beschichtungsprozesses in diesen Bereichen nicht aufgetragen wird. Hierfür können beispielsweise Schablonen verwendet werden. Anschließend wird die Abdeckung entfernt. Alternativ kann die Beschichtung auch zunächst auf die gesamte Oberfläche der Endoprothese (einschließlich der ggf. vorgesehenen Degradationselemente) aufgebracht werden und anschließend in den Degradationsbereichen mindestens teilweise entfernt werden. Bei einem am Ende 23 eines fingerförmigen Fortsatzes 22 angeordneten Degradationsbereich kann dies beispielsweise dadurch geschehen, dass ein Teil dieses Endes abgeschnitten wird.

Die Strukturformeln von Parlyene C und Parylene N, welche jeweils ein bevorzugtes Material der Beschichtung 30 darstellen, sind in Figur 4a bzw. in Figur 4b gezeigt.

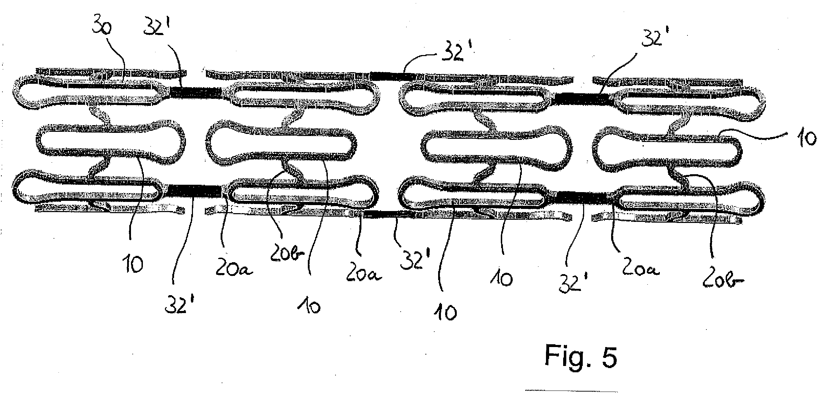

In Figur 5 ist noch einmal ein längerer Abschnitt einer erfindungsgemäßen Endoprothese in Form eines Stents dargestellt, die an den in Längsrichtung verlaufenden Verbindungsstegen 20a der Stützelemente 10 ringförmige und umlaufende Aussparungen 32' in der Beschichtung 30 aufweisen, die sich fast über die gesamte Länge dieser Verbindungsstege 20a erstrecken. Im Bereich dieser, in Figur 5 schwarz gekennzeichneten Aussparungen liegt das degradierbare Material frei. Die nicht in Längsrichtung sondern geschwungen in im Wesentlichen radialer Richtung verlaufenden Verbindungsstege 20b der Stützelemente 10 weisen keine Aussparungen auf.

### Bezugszeichenliste:

- 10: Stützelement
- 20, 20a, 20b: Verbindungssteg
- 22: fingerförmiger Fortsatz
- 23: wegragendes Ende des fingerförmigen Fortsatzes 22
- 25: kreisrunder Bereich
- 30: Beschichtung
- 32, 32': ringförmige Aussparung

## Patentansprüche

1. Intraluminale Endoprothese, z.B. ein Stent, mit einem Grundgitter bestehend aus einem zumindest weitestgehend biodegradierbaren Material und einer auf dem biodegradierbaren Material angeordneten Beschichtung (30), wobei das biodegradierbare Material Mg oder eine Mg-Legierung enthält, wobei die Beschichtung (30) inert ausgebildet ist und dass das Grundgitter vollständig durch die Beschichtung (30) bis auf mindestens einen Degradationsbereich (23, 25, 32, 32') zur gezielten Steuerung der Degradation in diesem Bereich bedeckt ist, wobei der mindestens eine Degradationsbereich (23, 25, 32, 32') als Aussparung in der inerten Beschichtung (30) ausgebildet ist, **dadurch gekennzeichnet dass**,
eine Vielzahl von Degradationsbereichen (23, 25, 32, 32') lediglich im Bereich der Verbindungsstege (20, 20a) angeordnet ist.

2. Endoprothese nach Anspruch 1, **dadurch gekennzeichnet, dass** die inerte Beschichtung (30) zusätzlich flexibel ausgebildet ist.

3. Endoprothese nach einem der Ansprüche 1 oder 2, **dadurch gekennzeichnet, dass** die inerte Beschichtung (30) eine oder mehrere Verbindungen der Gruppe bestehend aus Polysulfon, Silikonkautschuk, Polyurethan, synthetisches Glycocalix, amorphes Siliziumcarbid, Diamond Like Carbon (DLC), Magnesiumphosphat sowie Magnesiumoxid und deren Mischverbindungen aufweist.

4. Intraluminale Endoprothese, z.B. ein Stent, mit einem Grundgitter bestehend aus einem zumindest weitestgehend biodegradierbaren Material und einer auf dem biodegradierbaren Material angeordneten Beschichtung (30), wobei das biodegradierbare Material Mg oder eine Mg-Legierung enthält, wobei die Beschichtung (30) Parylene enthält, vorzugsweise mindestens überwiegend Parylene, besonders bevorzugt Parylene C oder Parylene N, enthält, und dass das Grundgitter vollständig durch die Beschichtung (30) bis auf mindestens einen Degradationsbereich (23, 25, 32, 32') zur gezielten Steuerung der Degradation in diesem Bereich bedeckt ist, wobei der mindestens eine Degradationsbereich (23, 25, 32, 32') als Aussparung in der Parylene enthaltenden Beschichtung (30) ausgebildet ist, **dadurch gekennzeichnet dass**,
eine Vielzahl von Degradationsbereichen (23, 25, 32, 32') lediglich im Bereich der Verbindungsstege (20, 20a) angeordnet ist.

5. Endoprothese nach Anspruch 4, **dadurch gekennzeichnet, dass** die Schichtdicke der Parylene enthaltende Beschichtung (30) in den von den Degradationsbereichen (23, 25, 32, 32') verschiedenen Bereichen zwischen etwa 0,1 µm und etwa 10 µm, bevorzugt zwischen etwa 0,4 µm und etwa 7 µm, besonders bevorzugt zwischen etwa 1 µm und etwa 5 µm beträgt.

6. Endoprothese nach einem der vorhergehenden Ansprüche, **dadurch gekennzeichnet, dass** zwischen der inerten Beschichtung oder der Parylene enthaltenden Beschichtung (30) und dem Material des Grundgitters eine Haftvermittlerschicht angeordnet ist, die vorzugsweise eine oder mehrere Verbindungen der Gruppe Magnesiumoxid, Magnesiumphosphat, anorganische Magnesiumverbindungen enthält.

7. Endoprothese nach einem der vorhergehenden Ansprüche, **dadurch gekennzeichnet, dass** das Grundgitter mindestens ein Degradationselement (22) aufweist, das von dem Grundgitter im Wesentlichen fortsatzartig wegragt und mindestens einen Degradationsbereich (23) aufweist und/oder dass das Degradationselement (22) im Wesentlichen fingerförmig ausgebildet ist und/oder dass sich das Degradationselement (22) im Wesentlichen im Bereich des durch das Grundgitter ausgebildeten Mantelvolumens erstreckt.

8. Endoprothese nach Anspruch 7, **dadurch gekennzeichnet, dass** jedes Degradationselement (22) genau einen Degradationsbereich (23) aufweist, der an dem Ende des Degradationselements angeordnet ist, das von dem Grundgitter wegragt.

9. Endoprothese nach Anspruch 7 oder 8, **dadurch gekennzeichnet, dass** das Degradationselement (22) einen Durchmesser von etwa 50 bis etwa 200 µm aufweist und/oder dass das Degradationselement (22) eine Länge von bis zu etwa 0,3 mm, vorzugsweise von bis zu etwa 0,1 mm aufweist.

10. Endoprothese nach einem der vorhergehenden Ansprüche, **dadurch gekennzeichnet, dass** der mindestens eine Degradationsbereich (23, 25, 32) als ringförmige Aussparung (32, 32') ausgebildet ist, die sich um ein Verbindungssteg (20, 20a) herum erstreckt und/oder dass der mindestens eine Degradationsbereich (23, 25, 32, 32') als kreisrunde oder mehreckige Aussparung (25) in der Beschichtung (30) ausgebildet ist, welche vorzugsweise einen Durchmesser von etwa 1 bis 10 µm aufweist.

11. Endoprothese nach einem der vorhergehenden Ansprüche, **dadurch gekennzeichnet, dass** das Grundgitter in axialer Richtung hintereinander angeordnete, im Wesentlichen in Umfangsrichtung verlaufende Stützelemente (10) und die einzelnen Stützelemente verbindenden Verbindungsstege (20, 20a, 20b) aufweist, wobei mindestens ein Degradationsbereich (23, 25, 32, 32') an den an einem dieser Verbindungsstege (20) angeordneten Degradationselementen (22) vorgesehen ist.

12. Endoprothese nach einem der vorhergehenden Ansprüche, **dadurch gekennzeichnet, dass** das biodegradierbare Material WE43enthält.

13. Endoprothese nach einem der Ansprüche 1 bis 10, **dadurch gekennzeichnet, dass** die inerte oder die Parylene enthaltende Beschichtung (30) zusätzlich eine oder mehrere Polymere der Gruppe bestehend aus Polyestern, vorzugsweise Polylactiden, und Polypeptiden aufweist.

14. Verfahren zur Herstellung einer Endoprothese nach einem der Ansprüche 4 bis 13, **gekennzeichnet durch** die folgenden Schritte:
- Bereitstellen des Grundgitters der Endoprothese ggf. mit Degradationselementen
- Aufbringen einer Parylene enthaltenden Beschichtung auf die Oberfläche der Endoprothese derart, dass diese vollständig bedeckt ist, vorzugsweise mittels Gasphasenabscheidung und
- Durchführung einer Sauerstoffplasmabehandlung.

15. Verfahren nach Anspruch 14, **dadurch gekennzeichnet, dass** eine etwa 0,1 bis etwa 10 µm, vorzugsweise etwa 0,4 µm bis etwa 7 µm, besonders bevorzugt etwa 1 µm bis etwa 5 µm dicke Parylene enthaltende Beschichtung aufgebracht wird.

## Claims

1. An intraluminal endoprosthesis, for example a stent, having a basic mesh consisting of an at least largely biodegradable material and a coating (30) arranged on the biodegradable material, wherein the biodegradable material contains Mg or an Mg alloy, wherein the coating (30) is inert and the basic mesh is completely covered by the coating (30) apart from at least a degradation area (23, 25, 32, 32') for purposeful control of the degradation in this area, wherein the at least one degradation area (23, 25, 32, 32') is formed as a recess in the inert coating (30), **characterised in that** a plurality of degradation areas (23, 25, 32, 32') is arranged only in the area of the connecting webs (20, 20a).

2. The endoprosthesis according to claim 1, **characterised in that** the inert coating (30) is additionally flexible.

3. The endoprosthesis according to any one of claims 1 or 2, **characterised in that** the inert coating (30) comprises one or more compounds from the group consisting of polysulfone, silicone rubber, polyurethane, synthetic glycocalix, amorphous silicon carbide, diamond-like carbon (DLC), magnesium phosphate, magnesium oxide and mixtures thereof.

4. An intraluminal endoprosthesis, for example a stent, having a basic mesh consisting of an at least largely biodegradable material and a coating (30) arranged on the biodegradable material, wherein the biodegradable material contains Mg or an Mg alloy, wherein the coating (30) contains parylene, preferably at least predominantly parylene, particularly preferably parylene C or parylene N, and the basic mesh is completely covered by the coating (30) apart from at least a degradation area (23, 25, 32, 32') for purposeful control of the degradation in this area, wherein the at least one degradation area (23, 25, 32, 32') is formed as a recess in the parylene-containing coating (30), **characterised in that**
a plurality of degradation areas (23, 25, 32, 32') is arranged only in the area of the connecting webs (20, 20a).

5. The endoprosthesis according to claim 4, **characterised in that** the layer thickness of the parylene-containing coating (30) in the areas different from the degradation areas (23, 25, 32, 32') is between approximately 0.1 µm and approximately 10 µm, preferably between approximately 0.4 µm and approximately 7 µm, particularly preferably between approximately 1 µm and approximately 5 µm.

6. The endoprosthesis according to any one of the preceding claims, **characterised in that** an adhesion-promoting layer is arranged between the inert coating or the parylene-containing coating (30) and the material of the basic mesh, which adhesion-promoting layer preferably contains one or more compounds from the group comprising magnesium oxide, magnesium phosphate and inorganic magnesium compounds.

7. The endoprosthesis according to any one of the preceding claims, **characterised in that** the basic mesh comprises at least one degradation element (22) which protrudes substantially away from the basic mesh and has at least one degradation area (23), and/or **in that** the degradation element (22) has substantially a finger shape and/or **in that** the degradation element (22) extends substantially in the area of the jacket volume formed by the basic mesh.

8. The endoprosthesis according to claim 7, **characterised in that** each degradation element (22) has exactly one degradation area (23) which is arranged on the end of the degradation element that protrudes away from the basic mesh.

9. The endoprosthesis according to claim 7 or 8, **characterised in that** the degradation element (22) has a diameter of approximately 50 to approximately 200 µm, and/or **in that** the degradation element (22) has a length of up to approximately 0.3 mm, preferably of up to approximately 0.1 mm.

10. The endoprosthesis according to any one of the preceding claims, **characterised in that** the at least one degradation area (23, 25, 32) is formed as a ring-shaped recess (32, 32') which extends around a connecting web (20, 20a), and/or **in that** the at least one degradation area (23, 25, 32, 32') is formed as a circular or a polygonal recess (25) in the coating (30), which preferably has a diameter of approximately 1 to 10 nm.

11. The endoprosthesis according to any one of the preceding claims, **characterised in that** the basic mesh has supporting elements (10), are arranged axially in succession and run substantially in the circumferential direction, and connecting webs (20, 20a, 20b) that connect the individual supporting elements, wherein at least one degradation area (23, 25, 32, 32') is provided on the degradation elements (22) arranged on one of these connecting webs (20).

12. The endoprosthesis according to any one of the preceding claims, **characterised in that** the biodegradable material contains WE43.

13. The endoprosthesis according to any one of claims 1 to 10, **characterised in that** the inert coating or the parylene-containing coating (30) additionally comprises one or more polymers from the group consisting of polyesters, preferably polylactides and polypeptides.

14. A method for producing an endoprosthesis according to any one of claims 4 to 13, **characterised by** the following steps:
- providing the basic mesh of the endoprosthesis with degradation elements as appropriate;
- applying a parylene-containing coating to the surface of the endoprosthesis so that it is completely covered, preferably by means of vapour deposition; and
- treating the coating with oxygen plasma.

15. The method according to claim 14, **characterised in that** a parylene-containing coating approximately 0.1 to approximately 10 µm thick, preferably approximately 0.4 µm to approximately 7 µm thick, particularly preferably approximately 1 µm to approximately 5 µm thick is applied.

## Revendications

1. Endoprothèse intraluminale, par exemple, stent, avec un réseau de base constitué d'au moins un matériau largement biodégradable et d'un revêtement (30) disposé sur le matériau biodégradable, où le matériau biodégradable contient du Mg ou un alliage de Mg, où le revêtement (30) est conçu inerte et de sorte que le réseau de base est totalement recouvert par le revêtement (30) à l'exception d'au moins une zone de dégradation (23, 25, 32, 32') pour la conduite ciblée de la dégradation dans cette zone, où l'au moins une zone de dégradation (23, 25, 32, 32') est conçue sous la forme d'un endroit laissé libre dans le revêtement (30) inerte, **caractérisée en ce qu'**une multitude de zones de dégradation (23, 25, 32, 32') est simplement disposée dans la zone des connexions de liaison (20, 20a).

2. Endoprothèse selon la revendication 1, **caractérisée en ce que** le revêtement (30) inerte est en outre conçu souple.

3. Endoprothèse selon l'une des revendications 1 ou 2, **caractérisée en ce que** le revêtement (30) inerte présente un ou plusieurs composés du groupe constitué d'une polysulfone, d'un caoutchouc silicone, d'un polyuréthane, de glycocalyx synthétique, de carbure de silicium amorphe, de carbone de type diamant (DLC), de phosphate de magnésium ainsi que d'oxyde de magnésium et de leurs composés de mélange.

4. Endoprothèse intraluminale, par exemple, stent, avec un réseau de base constitué d'au moins un matériau largement biodégradable et d'un revêtement (30) disposé sur le matériau biodégradable, où le matériau biodégradable contient du Mg ou un alliage de Mg, où le revêtement (30) contient des parylènes, de préférence, au moins principalement des parylènes, plus préférentiellement du parylène C ou du parylène N, et que le réseau de base est totalement recouvert par le revêtement (30) à l'exception d'au moins une zone de dégradation (23, 25, 32, 32') pour une conduite ciblée de la dégradation dans cette zone, où l'au moins une zone de dégradation (23, 25, 32, 32') est conçue sous la forme d'un endroit laissé libre dans le revêtement (30) contenant des parylènes, **caractérisée en ce qu'**une multitude de zones de dégradation (23, 25, 32, 32') est disposée simplement dans la zone des connexions de liaison (20, 20a).

5. Endoprothèse selon la revendication 4, **caractérisée en ce que** l'épaisseur de couche du revêtement (30) contenant du parylène dans les diverses zones parmi les zones de dégradation (23, 25, 32, 32') se situe entre environ 0,1 µm et environ 10 µm, de préférence, entre environ 0,4 µm et environ 7 µm, de manière particulièrement préférée, entre environ 1µm et environ 5 µm.

6. Endoprothèse selon l'une des revendications précédentes, **caractérisée en ce qu'**une couche de promoteur d'adhésion est disposée entre le revêtement inerte, ou le revêtement (30) contenant des parylènes, et le matériau du réseau de base, qui contient de préférence un ou plusieurs composés du groupe oxyde de magnésium, phosphate de magnésium, composés inorganiques de magnésium.

7. Endoprothèse selon l'une des revendications précédentes, **caractérisée en ce que** le réseau de base présente au moins un élément de dégradation (22) qui dépasse du réseau de base essentiellement sous une forme saillante et présente au moins une zone de dégradation (23) et/ou que l'élément de dégradation (22) est conçu essentiellement sous la forme d'un doigt et/ou que l'élément de dégradation (22) s'étend essentiellement dans la zone du volume d'enveloppe formé par le réseau de base.

8. Endoprothèse selon la revendication 7, **caractérisée en ce que** chaque élément de dégradation (22) présente exactement une zone de dégradation (23) qui est disposée à l'extrémité de l'élément de dégradation qui dépasse du réseau de base.

9. Endoprothèse selon la revendication 7, ou 8, **caractérisée en ce que** l'élément de dégradation (22) présente un diamètre d'environ 50 à environ 200 µm et/ou que l'élément de dégradation (22) présente une longueur jusqu'à environ 0,3 mm, de préférence jusqu'à environ 0,1 mm.

10. Endoprothèse selon l'une des revendications précédentes, **caractérisée en ce que** l'au moins une zone de dégradation (23, 25, 32) est conçue sous la forme d'un endroit laissé libre (32, 32') en forme d'anneau qui s'étend autour d'une connexion de liaison (20, 20a) et/ou que l'au moins une zone de dégradation (23, 25, 32, 32') est conçue sous la forme d'un endroit laissé libre (25) en forme de cercle ou d'un polygone dans le revêtement (30), lequel présente un diamètre d'environ 1 à 10 µm.

11. Endoprothèse selon l'une des revendications précédentes, **caractérisée en ce que** le réseau de base présente des éléments de renfort (10) s'étendant dans la direction axiale, disposés les uns derrière les autres, essentiellement dans la direction circonférentielle et des connexions de liaison (20, 20a, 20b) reliant les éléments de renfort individuels, où au moins une zone de dégradation (23, 25, 32, 32') est prévue sur les éléments de dégradation (22) disposés sur l'une de ces connexions de liaison (20).

12. Endoprothèse selon l'une des revendications précédentes, **caractérisée en ce que** le matériau biodégradable contient du WE43.

13. Endoprothèse selon l'une des revendications 1 à 10, **caractérisée en ce que** le revêtement (30) inerte ou contenant les parylènes présente en outre un ou plusieurs polymères du groupe constitué de polyesters, de préférence de polylactides et de polypeptides.

14. Procédé de fabrication d'une endoprothèse selon l'une des revendications 4 à 13, **caractérisé par** les étapes suivantes :
- mise au point du réseau de base de l'endoprothèse, éventuellement avec des éléments de dégradation
- dépôt d'un revêtement contenant des parylènes sur la surface de l'endoprothèse de telle manière que celle-ci est totalement recouverte, de préférence, au moyen d'un dépôt chimique en phase vapeur, et
- réalisation d'un traitement par un plasma d'oxygène.

15. Procédé selon la revendication 14, **caractérisé en ce qu'**un revêtement contenant des parylènes d'une épaisseur d'environ 0,1 à environ 10 µm, de préférence d'environ 0,4 µm à environ 7 µm, de manière particulièrement préférée d'environ 1 µm à environ 5 µm est déposé.
